# EUROPÄISCHE PATENTSCHRIFT

(11) **EP 2 889 047 B1**
(45) Veröffentlichungstag und Bekanntmachung des Hinweises auf die Patenterteilung: **05.12.2018**
(21) Anmeldenummer: 14200064.5
(22) Anmeldetag: 23.12.2014
(51) Int. Cl.: A61M 1/02

(54) **Steriles Blutbeutelsystem für die medizinische Entnahme und Kryokonservierung von Nabelschnurblut**
Sterile blood bag system for medical taking and cryopreserving of blood from an umbilical cord
Système de flacon de sang stérile pour le prélèvement médical et cryoconservation de sang de cordon ombilical

(30) Priorität: 27.12.2013 DE 102013227182
(43) Veröffentlichungstag der Anmeldung: 01.07.2015
(73) Patentinhaber: Evo3medica International GmbH, 04109 Leipzig (DE)
(72) Erfinder: Papavlassopoulos, Oliver, 04179 Leipzig (DE); Niescher, Susanne, 09599 Freiberg (DE)
(74) Vertreter: Kailuweit & Uhlemann Patentanwälte Partnerschaft mbB

(56) Entgegenhaltungen:
- EP-A1- 1 262 202
- WO-A1-03/086574
- WO-A1-2004/096319
- WO-A2-03/041634

## Beschreibung

Die vorliegende Erfindung betrifft ein in sich geschlossenes, steriles Blutbeutelsystem für die medizinische Entnahme und Kryokonservierung von Nabelschnurblut.

Nabelschnurblut (auch Plazentarestblut genannt) weist eine hohe Konzentration an Blutstammzellen (auch Hämatopoetische Stammzellen) auf, die normalerweise primär im Knochenmark, aber auch in der Leber und Milz vorkommen.

Zuerst wurden Blutstammzellen identifiziert, da sie die Zell-Linien des Blutes von Mäusen wiederherstellten, wobei die Mäuse ansonsten aufgrund einer letalen Strahlendosis gestorben wären. Nachfolgende Untersuchungen zeigten, dass Blutstammzellen unter bestimmten experimentellen Bedingungen zu ausgewählten Zelltypen differenzieren können, die nicht ausschließlich Bestandteile des Blutes sind. Ferner legen erste Experimente den Verdacht nahe, dass sich Blutstammzellen in eine Reihe von Zelltypen (bspw. Zellen der Skelett- oder Herzmuskulatur, des Gehirns) differenzieren können, die sich im Anschluss ihrer Differenzierung in das entsprechende Gewebe integrieren.

Die große Anzahl vielversprechender Experimente, die einen Einblick in die Möglichkeiten der gezielten Differenzierung von Blutstammzellen bieten, lassen die Aussichten für therapeutische Anwendungen mit adulten Stammzellen sehr positiv erscheinen. Adulte Stammzellen sind allerdings sehr selten, schwer zu identifizieren und noch schwerer von den anderen Zellen der jeweiligen Organe zu isolieren.

Im Gegensatz dazu stellt Nabelschnurblut, das direkt nach der Abnabelung des Kindes gesammelt und konserviert wird, einen reichhaltigen Pool undifferenzierter Stammzellen dar, die potentiell für parentale Therapien zur Verfügung stehen. Angesichts des enormen Potentials von undifferenzierten Blutstammzellen für zukünftige parentale Therapien kommt dabei der Kryokonservierung von Nabelschnur-Vollblut eine immense wirtschaftliche Bedeutung zu.

Um Stammzellpräparate für Patienten monate- bis jahrelang haltbar zu machen, ist eine Weiterverarbeitung des entnommenen Nabelschnurbluts, bevorzugt direkt nach der Entnahme erforderlich. Unter dem Begriff der "Weiterverarbeitung" bezeichnet der Fachmann die Vorbereitung und Durchführung der Kryokonservierung und die Langzeitlagerung der Produktgemische in Kryotanks bis zu deren Verwendung.

Verfahren zur standardisierten Kryokonservierung von Vollblutpräparaten (im Speziellen Nabelschnurblut) gehören zum Stand der Technik und erfolgen entsprechend der gesetzlichen Richtlinien. Die etablierten Verfahren zur Vorbereitung der Kryokonservierung und Weiterverarbeitung von Nabelschnurblut werden zum gegenwärtigen Zeitpunkt in das sog. "offene" System und das "geschlossene" System unterteilt.

Beide Systeme bedingen allerdings, dass die Weiterverarbeitung des entnommenen Nabelschnurblutes grundsätzlich zentralisiert und unter höchsten Qualitäts- und Sicherheitsstandards in geforderten Reinräumen der Klassen A bis D erfolgen muss. Zur Aufrechterhaltung dieser Reinraumklassen und um einen sterilen Prozessablauf zu garantieren erfolgt arbeitstäglich ein umfangreiches, zeitaufwendiges und dadurch kostenintensives Hygienemonitoring des Personals und der gesamten Räumlichkeiten.

Beim *"offenen"* System werden folgende Schritte durchgeführt: Während der Entnahme des Nabelschnurbluts wird dieses direkt in einen ersten O₂-permeablen Entnahmebeutel (sog. Transportbeutel) eingeleitet und mit einer Menge an Zitrat als Antikoagulanz (bspw. CPD) gemischt. Das Zitrat wird dazu durch eine externe Zugabe in den ersten Entnahmebeutel eingeleitet, oder liegt bereits vom Werk her im ersten Beutel vor.

Hierzu beschreibt bspw. US 5,356,373 einen einzelnen Entnahmebeutel in Verbindung mit einem *"offenen"* System, in dem CPDA als Antikoagulanz bereits in dem Entnahmebeutel vorgelegt ist.

Im Anschluss wird der Entnahmebeutel unter Einhaltung der Transporttemperatur (18-22°C) innerhalb von max. 48 h in ein Zentrallabor mit integriertem Reinstraum verbracht. Es muss jedoch eine deutliche Verkürzung der Transportzeit anzustreben sein, da bereits während der ersten 36 h signifikante Zellschädigungen auftreten. Vor Ort wird der Entnahmebeutel geöffnet und dem Nabelschnurblut wird Dimethylsulfoxid (DMSO) als Kryoprotektivum zugesetzt. Nach der Qualitätskontrolle erfolgt die Überführung des Präparats in einen neuen temperaturstabilen Einfrierbeutel, so dass die Kryokonservierung des Präparats vorzugsweise in der Gasphase von flüssigem Stickstoffs als Kühlmittel bei ca. -140°C durchgeführt werden kann.

Bei Verfahren die nach dem "offenen" System ablaufen, ist insbesondere nachteilig, dass das System durch das Öffnen des Entnahmebeutels in sich nicht mehr steril ist, wodurch Reinstraumbedingungen für die Weiterverarbeitung erforderlich sind.

Aus US 5,879,318 A und EP 1 438 088 A1 sind hingegen "geschlossene" Beutelsysteme bekannt, die sowohl einen O₂-permeablen Entnahmebeutel als auch einen Einfrierbeutel aufweisen, wobei die beiden Beutel über Verbindungsleitungen miteinander verbunden sind, so dass der teure und aufwändig zu betreibende Reinraum entfällt.

Etablierte "geschlossene" Systeme haben jedoch den Nachteil, dass die Überführung des Blutes aus dem Entnahmebeutel in den Einfrierbeutel manuell erfolgen muss, wobei die Überführung sehr zeitintensiv ist und zugleich wertvolles Nabelschnurblut im Entnahmebeutel und dem Verbindungssystem verbleibt.

WO 03/041634 A2 (VITA 34 AG) betrifft ein Beutelsystem für die Kryokonservierung von Körperflüssigkeiten, insbesondere Blut, Knochenmark oder Nabelschnurblut. Das Beutelsystem umfasst eine Vorrichtung zur Entnahme der Körperflüssigkeiten, einen oder mehrere Bereiche zum Mischen der Körperflüssigkeiten mit Flüssigkeiten zur Vermeidung von Blutgerinnung oder zur Kryokonservierung, mehrere Absperrorgane und Verbindungsleitungen und ein Ein- und/oder Auslassorgan.

Es besteht daher ein großer Bedarf, ein "geschlossenes" Beutelsystem für die Entnahme und Kryokonservierung von Nabelschnurblut zur Verfügung zu stellen, bei dem die Weiterverarbeitung des entnommenen Nabelschnurblutes dezentral erfolgen kann und der zeitliche als auch arbeitsintensive Vorbereitungsaufwand für die Einlagerung minimiert wird, wodurch gleichfalls die Verluste an entnommenen Nabelschnurblut während der Weiterverarbeitung entfallen.

Erfindungsgemäß wird die Aufgabe durch ein geschlossenes, in sich steriles Blutbeutelsystem für die Entnahme und Kryokonservierung von Nabelschnurblut gelöst, das gleichzeitig zur Einlagerung genutzt werden kann und das als Komponenten zumindest
- ein Punktionsstück (1),
- einen einfriertauglichen Entnahmebeutel, wobei der Einfrierbeutel ein kombinierter Misch- und Einfrierbeutel (2) ist und
- ein Einlassorgan (4)
aufweist, wobei die einzelnen Komponenten des Blutbeutelsystems untereinander kommunizierend über Verbindungsleitungen (8) miteinander so verbunden sind, dass der kombinierte Misch- und Einfrierbeutel zur direkten Aufnahme von Nabelschnurblut während dessen Entnahme dient und gleichzeitig zur Kryokonservierung nutzbar ist, dass die einzelnen Komponenten des Blutbeutelsystems durch Trennvorrichtungen (5) steril voneinander separierbar sind, dass dem Punktionsstück (1) und dem Misch- und Einfrierbeutel (2) ein Reservoirbereich (3) zwischengeordnet ist, wobei der Reservoirbereich (3) an seinen gegenüberliegenden Seiten jeweils eine Öffnung, einen Ein- und Auslass, aufweist, wobei der Reservoirbereich (3) in die Hauptverbindungsleitung (12), die das Punktionsstück (1) und den kombinierten Misch- und Einfrierbeutel (2) verbindet, direkt und gleichgerichtet integriert ist.

Vorteilhaft entfällt durch die Nutzung des erfindungsgemäßen einfriertauglichen Entnahmebeutels (kombinierten Misch- und Einfrierbeutel) die zeitaufwändige und dadurch kostenintensive Überführung des Nabelschnurblutes zwischen einem getrennten Entnahmebeutel und einem oder mehreren Einfrierbeuteln, wodurch die Handhabung bei der Weiterverarbeitung des entnommenen Nabelschnurblutes vereinfacht und das Risiko der Kontamination des Einlagerungspräparates stark minimiert wird.

Besonders vorteilhaft hat sich gezeigt, dass bei dem erfindungsgemäßen Blutbeutelsystem mit einem zwischengeordneten Reservoirbereich (3) die Koagulation des Blutes und somit bspw. Rückstände in den Verbindungsleitungen bzw. den einzelnen Komponenten des erfindungsgemäßen Blutbeutelsystems minimiert werden.

Das erfindungsgemäße Beutelsystem hat gegenüber den bekannten "geschlossenen" Systemen zudem den Vorteil, dass nur ein einziger Beutel für die Entnahme und Kryokonservierung von Nabelschnurblut erforderlich ist und zudem zusätzliche Verbindungsleitungen entfallen. Aufgrund der Einsparung von Material werden zum einen die Produktionskosten für ein erfindungsgemäßes Beutelsystem minimiert und noch wichtiger wird der anfallende Klinikmüll um ca. 80% reduziert.

Vorangegangene und nachfolgende Quellennachweise sind nur insoweit aufgeführt, als dass es für das Verständnis der Erfindung für den Fachmann notwendig ist.

Das erfindungsgemäße sterile Blutbeutelsystem ist ein "geschlossenes" Einbeutelsystem, dessen einzelne Komponenten durch Verbindungsleitungen kommunizierend miteinander verbunden sind, wobei sich die einzelnen Komponenten durch Trennvorrichtungen (5) steril voneinander separieren lassen.

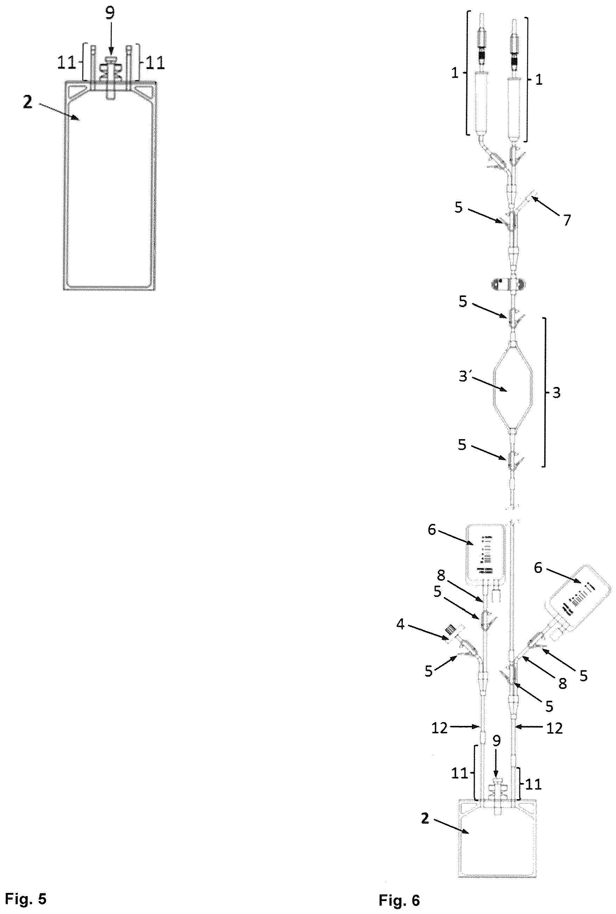

Erfindungsgemäß dient mindestens ein Punktionsstück (1, Fig. 6) zur Überführung des Nabelschnurblutes aus der Nabelschnur und/oder der Plazenta in das Blutbeutelsystem (d. h. Entnahme) bspw. durch gezielte Punktion(en) der Nabelschnur nach der Abnabelung des Kindes. Beispielsweise ist das Punktionsstück (1) eine Entnahmekanüle aus Kunststoff oder Edelstahl. Vorteilhaft weist das Punktionsstück einen inneren Durchmesser von 1,9 bis maximal 2,5 mm auf. Besonders vorteilhaft weist das Punktionsstück eine zusätzliche rückwandige Öffnung (back-eye) auf, um eine bessere Ausbeute an Zellen bzw. Präparat zu erzielen. Bevorzugt ist das Punktionsstück durch eine integrierte Verschlusskappe geschützt, um eine Kontamination des Präparates und Verletzungsgefahr des Anwendungspersonals zu minimieren. Besonders bevorzugt kann das Punktionsstück nach Verwendung durch eine weitere kombinierte (bspw. fest verbundene oder nachträglich aufzubringende) Schutzkappe gesichert werden.

Im Sinne der Erfindung wird unter der Bezeichnung *"Entnahmebeutel"* ein autarker Aufnahmebereich für Flüssigkeiten verstanden, der durch einen Beutelkörper abgegrenzt ist. Der Beutelkörper wird durch Beutelwände gebildet. Bevorzugt bestehen die Beutelwände aus einem flexiblen und elastischen Organopolymer. Zweckdienlich aber nicht notwendigerweise ist mindestens eine Beutelwand des Beutelkörpers transparent (d. h. durchsichtig), da dadurch vorteilhaft eine optische Begutachtung des entnommenen Nabelschnurblutes nach der Entnahme und während der einzelnen Schritte der Weiterverarbeitung erfolgen kann. Der Entnahmebeutel ist entweder als Einkammerbeutel oder Mehrkammerbeutel ausgebildet.

Unter der Bezeichnung *"kombinierter Misch- und Einfrierbeutel"* (2) wird erfindungsgemäß ein Beutel verstanden, der zur direkten Aufnahme von Nabelschnurblut während dessen Entnahme dient und der gleichzeitig zur Kryokonservierung des entnommenen Nabelschnurbluts genutzt werden kann.

Bevorzugt ist der kombinierte Misch- und Einfrierbeutel (2) ein Einkammerbeutel, wodurch vorteilhaft eine gegebenenfalls zeit- oder arbeitsintensive Überführung des Nabelschnurbluts von einer Kammer in die andere entfällt.

In einer alternativ bevorzugten Ausgestaltung der Erfindung ist der kombinierte Misch- und Einfrierbeutel ein Mehrkammersystem. Dies ist insbesondere vorteilhaft in Fällen, bei denen ein größeres Volumen an Nabelschnur entnommen wird, sodass optional ein Anteil des entnommenen Nabelschnurblutes in das zweite System geleitet werden kann.

Ein kombinierter Misch- und Einfrierbeutel (2) weist bevorzugt maximal zwei Einlässe (11) und ein Auslassorgan (9) für Flüssigkeiten (bspw. Nabelschnurblut, Kryoprotektivum) auf. Bevorzugt ist der Misch- und Einfrierbeutel (2) über die Einlässe und daran gekoppelte Hauptverbindungsleitungen kommunizierend mit dem Punktionsstück (1) und dem Einlassorgan (4) des erfindungsgemäßen Blutbeutelsystems verbunden.

Bevorzugt bestehen die Beutelwände des kombinierten Misch- und Einfrierbeutels aus einem kältebeständigen Organopolymer, besonders bevorzugt aus Polyurethan (DIN-Kurzzeichen PUR) oder Ethylvinylacetat-basierten Elastomer (DIN-Kurzzeichen EVA), ganz besonders bevorzugt aus einem Polyurethan-Elastomer. Das Organopolymer ist bevorzugt flexibel genug, um den physikalischen Beanspruchungen einer Kryokonservierung und der nachgeführten Verarbeitung bei der Entnahme aus den Kryotanks standzuhalten.

Bevorzugt bestehen die Einlässe (11) und das Auslassorgan (9) des kombinierten Misch-und Einfrierbeutels aus einem kältebeständigen Organopolymer, besonders bevorzugt aus Polyurethan (DIN-Kurzzeichen PUR) oder Ethylvinylacetat-basierten Elastomer (DIN-Kurzzeichen EVA), ganz besonders bevorzugt aus einem Polyurethan-Elastomer. Das Organopolymer ist bevorzugt flexibel genug, um den physikalischen Beanspruchungen einer Kryokonservierung und der nachgeführten Verarbeitung bei der Entnahme aus den Kryotanks standzuhalten.

Vorteilhaft zeichnen sich Polyurethan-Elastomere und Ethylvinylacetat-basierte Elastomere neben einer ausgeprägten mechanischen Festigkeit, bspw. einer Zugfestigkeit im Bereich zwischen 15 und 40 MPa und einer Reißdehnung im Bereich zwischen 300 und 500% (beide bestimmt nach DIN EN ISO 527) und einer ausgeprägten Tieftemperaturbeständigkeit (sehr gute Kälteflexibilität) auch durch eine gute Mikrobenbeständigkeit aus. Spezielle Polyurethan-Materialien sind dafür bekannt, den physikalischen Beanspruchungen während der Einlagerungs- und Wiederauftauprozesse in der Gasphase von flüssigem Stickstoff standzuhalten. Besonders bevorzugt weist ein kältebeständiges Organopolymer eine O₂-Permeabilität im Bereich zwischen 1500 und 2500 cm³ pro m² Organopolymer auf, wodurch vorteilhaft die Zellvitalität nach der Entnahme des Nabelschnurblutes erhalten bleibt. Gleichzeitig weisen die kältebeständigen Organopolymere eine geringe N₂-Permeabilität auf.

Während des Prozesses der Kryokonservierung durchlaufen die kältebeständigen Organopolymere innerhalb kürzester Zeit einen Temperaturbereich von 23°C bis -140°C. Beutelwände aus Polyurethan-Elastomeren weisen daher hohe Verschleiß- und Abriebfestigkeiten auf. Vorteilhaft ist das Organopolymer chemisch stabil (d.h. unreaktiv während des vorgesehenen Gebrauchs, unter Erhalt seiner Eigenschaften), sodass sich vom Zeitpunkt der Entnahme des Nabelschnurblutes bis zur Kryokonservierung bzw. dem Zeitraum nach dem Auftauen bis zur Verwendung des Blutpräparates keine zellschädigenden Verbindungen daraus lösen.

In einer bevorzugten Ausgestaltung der Erfindung sind die Beutelwände des kombinierten Misch- und Einfrierbeutels (2) daher aus einem kältebeständigen Organopolymer, wobei das kältebeständige Organopolymer eine O₂-Permeabilität im Bereich zwischen 1500 und 2500 cm³ pro m² Organopolymer aufweist.

Bevorzugt weist eine Kammer des Misch- und Einfrierbeutel (2) ein maximales Hohlvolumen im Bereich zwischen 10 bis 200 mL, besonders bevorzugt zwischen 20 bis 150 mL auf. Dadurch ist der Misch- und Einfrierbeutel vorteilhaft zur Aufnahme des gesamten entnommenen Nabelschnurblutes geeignet.

Besonders vorteilhaft zeichnet sich ein kombinierter Misch- und Einfrierbeutel (2) durch die Wahl von Polyurethan als Organopolymer, speziell das Polyurethan-Elastomer, durch eine hohe Elastizität und Temperaturbeständigkeit, bevorzugt im Bereich von -196 bis 70°C aus. Bevorzugt ist ein kombinierter Misch- und Einfrierbeutel im Sinne der Erfindung aus einem kältestabilen Organopolymer (Stabilität mind. bis -140°C oder auch -196°C) gefertigt, welches gleichzeitig eine genügend hohe O₂-Permeabilität aufweist, um die Vitalität der Blutzellen über den Verarbeitungszeitraum bis hin zur Kryokonservierung gewährleisten zu können.

Besonders bevorzugt besitzen die Einlässe des kombinierten Misch- und Einfrierbeutels (11) eine Länge im Bereich von wenigstens 60 bis maximal 90 mm, um das Anfertigen von Aliquotproben für die Einlagerung zu ermöglichen.

Vorteilhaft ist das Auslassorgan (9) des kombinierten Misch- und Einfrierbeutels (2) ein hermetisch dichtes Organ, welches sich nach vollendeter Einlagerung leicht öffnen lässt, so dass das eingelagerte Präparat anschließend leicht zugänglich und entnehmbar ist.

*"Hermetisch dicht"* im Sinne der Erfindung bedeutet, dass weder ein Austreten von gasförmigen oder flüssigen Medien aus dem Misch- und Einfrierbeutel (2), noch ein Eindringen von Medien aus der Umgebung, möglich ist.

*"Mehrkammerbeutel"* bedeutet im Sinne der Erfindung, dass das freie verfügbare Volumen eines Beutels durch den Einzug von Trennvorrichtungen in separate Bereiche (d. h. Kammern) getrennt ist, wobei der Fachmann unter der Bezeichnung *"Einkammerbeutel"* einen Beutel versteht, der keine Trennvorrichtungen aufweist. Eine Trennvorrichtung kann hierin bspw. eine Trennvorrichtung aus einem starren, brechfähigen, Material oder alternativ bevorzugt aus einem flexiblen Material sein. Besonders bevorzugt ist die Trennvorrichtung aus einem flexiblen Organopolymer, wodurch vorteilhaft die Handhabung für medizinisches Personal erleichtert wird, da durch manuelle Druckeinwirkung auf die flexiblen Trennvorrichtungen der Flüssigkeits- und Gasaustausch zwischen den Kammern innerhalb des Beutels bewirkt werden kann. Weiterhin können die einzelnen Kammern über (trennbare) Zuleitungen miteinander verbunden sein.

Erfindungsgemäß dient ein kombinierter Misch- und Einfrierbeutel zur Aufnahme sowie Aufbewahrung von Nabelschnurblut und/oder eines Nabelschnurblutpräparates. Bevorzugt lässt sich der kombinierter Misch- und Einfrierbeutel vollständig und eigenständig von dem Beutelsystem abtrennen und/oder es können einzelne Komponenten von dem kombinierten Misch- und Einfrierbeutel abgetrennt werden.

Bevorzugt aber nicht essentiell kann ein Aufnahmebereich des Beutels vor der Aufnahme des Nabelschnurbluts und/oder eines Nabelschnurblutpräparates Flüssigkeiten und/oder Feststoffe enthalten, die die Kryokonservierung unterstützen.

Unter dem maximalen Hohlvolumen eines Beutels oder eines Volumenkörpers versteht der Fachmann den freien räumlichen Inhalt eines Körpers (d. h. das Fassungsvermögen) innerhalb des entsprechenden Körpers.

Der Begriff *"zwischengeordneter Reservoirbereich"* (3) bezeichnet im Sinne der Erfindung einen Volumenbereich, der zwischen dem Punktionsstück (1) und dem kombinierten Misch-und Einfrierbeutel (2) angeordnet ist. Bevorzugt aber nicht notwendigerweise enthält der Reservoirbereich eine Flüssigkeit, die die Kryokonservierung ermöglicht und/oder unterstützt, bspw. das Antikoagulanz zur Zellstabilisierung, das Kryoprotektivum und/oder eine Verdünnungsflüssigkeit, besonders bevorzugt ein Antikoagulanz wie Heparin oder EDTA ganz besonders bevorzugt ein Zitrat.

Bevorzugt ist das Antikoagulanz ausgewählt aus der Gruppe Zitratdextrose, Zitratphosphatdextose (CPD), Zitratphosphatdextrose-adenin (bspw. CPDA), Natriumzitrat oder einer Kombination dieser (bspw. Acid-Citrat-Dextrose). Insbesondere bevorzugt ist das Zitrat CPD.

Bevorzugt weist der zwischengeordnete Reservoirbereich (3) ein Hohlvolumen (Fassungsvermögen) in einem Bereich zwischen 10 bis maximal 30 mL, bevorzugt zwischen 15 und 25 mL auf und ist dabei insbesondere nicht ausreichend, um die Gesamtmenge des entnommenen Nabelschnurblutes aufzunehmen.

Der Reservoirbereich (3) ist so konzipiert, dass der Reservoirbereich (3) an seinen gegenüberliegenden Seiten jeweils eine Öffnung (d. h. einen Ein- und einen Auslass) aufweist, wodurch der Reservoirbereich (3) mit den anderen Komponenten eines erfindungsgemäßen Blutbeutelsystems über Verbindungsleitungen kommunizierend verbunden ist. Der Reservoirbereich (3) ist direkt und gleichgerichtet in den Verlauf der Hauptverbindungsleitung (12) integriert, die das Punktionsstück (1) mit dem kombinierten Misch- und Einfrierbeutel (2) verbindet.

Das hat insbesondere den Vorteil, dass das Nabelschnurblut während des Entnahmevorgangs direkt durch den Reservoirbereich (3) geleitet wird. Dabei kann es bspw. vorteilhaft sein, dass das Antikoagulanz in dem Hohlvolumen des Reservoirbereichs (3) erst unmittelbar vor der Entnahme des Nabelschnurblutes aus dem Reservoirbereich (3) durch die Verbindungsleitung in den Misch- und Einfrierbeutel (2) eingeleitet wird, wodurch die innere Wandung der Verbindungsleitung und des Misch- und Einfrierbeutels (2) mit dem Antikoagulanz benetzt wird.

Eine solche unmittelbare Benetzung der inneren Wandung der Verbindungsleitung, insbesondere der Hauptverbindungsleitung (12) und des Misch- und Einfrierbeutel (2) mit dem Antikoagulanz hat den Vorteil, dass das Auftreten von Verklumpungen und Rückstände von Nabelschnurblut (d.h. durch dessen Koagulation) bei der Entnahme verringert ist.

Vorteilhaft wird für den Stofftransport der Flüssigkeiten von dem Entnahmestück (1) zu dem Misch- und Einfrierbeutel (2) die Schwerkraft ausgenutzt. Zweckdienlich ist daher das Entnahmestück (1) in einer Ebene oberhalb des kombinierten Misch- und Einfrierbeutels (2) angeordnet.

In einer bevorzugten Ausgestaltung der Erfindung ist der zwischengeordnete Reservoirbereich (3) als Sachet (3') ausgebildet. Im Sinne der Erfindung ist ein Sachet (3') ein Volumenkörper aus einem Folienmaterial, der an seinen gegenüberliegenden Seiten jeweils eine Öffnung (d. h. einen Ein- und einen Auslass) aufweist. Bevorzugt weist ein Sachet (3') ein Hohlvolumen (Fassungsvolumen) im Bereich zwischen 10 bis maximal 30 mL, bevorzugt zwischen 15 und 25 mL auf.

Bevorzugt ist das Folienmaterial des Sachets ein thermoplastischer oder elastischer Organopolymer, insbesondere bevorzugt ein Polyolefin. Polyolefine sind beispielsweis Polyethylen (PE), Polypropylen (PP), Polyvinylchlorid (PVC), Polyester (wie PET oder PBT), Ethylenvinylacetat. Das Folienmaterial ist beständig gegenüber dem Antikoagulanz und beeinflusst die Zusammensetzung des Antikoagulanz über den Zeitraum der Produktlagerung nicht. Die Wasserdampfdurchlässigkeit des Folienmaterials liegt in einem Bereich zwischen 4,9 und 7,6 g/m² x d und ist so gewählt, dass in dem Antikoagulanz durch die gewählte Sterilisiationsmethode (bspw. Vakuum-Dampfautoklavierung) bzw. über den Zeitraum der definierten Produkthaltbarkeit (mindestens 12 Monate ab Herstellung) keine Konzentrationsveränderungen auftreten.

Es kann vorgesehen sein, dass das Sachet (3') im oberen Drittel, besonders bevorzugt im oberen Fünftel der Hauptverbindungsleitung (8) zwischen Punktionsstück (1) und kombiniertem Misch- und Einfrierbeutel (2), d. h. unmittelbar nach dem Punktionsstück (1), angeordnet ist. Eine derartige Anordnung des Sachets (3') in dem erfindungsgemäßen Blutbeutelsystem ist besonders vorteilhaft, wenn das Sachet eine Flüssigkeit, die die Kryokonservierung ermöglichen und/oder unterstützen, besonders bevorzugt das Antikoagulanz, aufweist, da das Antikoagulanz so erst unmittelbar vor der Entnahme des Nabelschnurblutes aus dem Sachet durch die Hauptverbindungsleitung in den Misch- und Einfrierbeutel geleitet wird, wodurch die innere Wandung der Hauptverbindungsleitung und des Misch- und Einfrierbeutels mit dem Antikoagulanz benetzt wird. Im Zusammenhang damit ist es besonders vorteilhaft, dass das Sachet an seinen Öffnungen (d. h. Ein- und Auslass) jeweils eine Trennvorrichtung aufweist, wodurch sichergestellt ist, dass die Flüssigkeiten, die die Kryokonservierung ermöglichen und/oder unterstützen, bis unmittelbar vor der Entnahme des Nabelschnurbluts in dem Sachet verbleiben.

Eine *"Hauptverbindungsleitung"* (12) ist im Sinne der Erfindung eine Verbindungsleitung, die den kombinierten Misch- und Einfrierbeutel über einen Einlass (11) einerseits direkt mit dem Punktionsstück (1) und/oder andererseits direkt mit dem Einlassorgan (4) des Blutbeutelsystems verbinden.

Eine Trennvorrichtung (5) ist bevorzugt als Abdrücksystem ausgebildet, das beispielsweise mittels äußerer Klammern (bspw. Herzklemmen) die Schläuche zwischen den Komponenten zur Abdichtung abdrückt und sich vor Anwendung öffnen lässt. Vorteilhaft erlaubt der Einsatz von Abdrücksystemen eine partikelfreie Abtrennung der einzelnen Komponenten untereinander. Um Undichtigkeiten nach der Abtrennung mit einem Abdrücksystem zu vermeiden, sind die Toleranzen der Schlauchdicke und des Abdrücksystems genau aufeinander abgestimmt.

Alternativ kann die Trennvorrichtung (5) auch als Trennbrechteil aus starren, brechfähigen Materialien ausgebildet sein. Trennbrechteile haben den Vorteil einer weitgehend universellen Verwendbarkeit, weisen jedoch insofern Nachteile auf, als sie nur eine begrenzte Brech- bzw. Mischöffnung freigeben oder es beim Aufbrechen des Trennbrechteils zu unerwünschter Partikelbildung kommen kann, die die Brech- bzw. Mischöffnung vollständig blockieren.

Vorzugsweise erfolgt die Zufuhr der Flüssigkeiten, die die Kryokonservierung ermöglichen und/oder unterstützen, bspw. des Kryoprotektivums und/oder eine Verdünnungsflüssigkeit über ein Einlassorgan (4), das eine Ausschlussgröße von maximal 500 *µ*m, besonders bevorzugt maximal 400 *µ*m, ganz besonders bevorzugt von maximal 250 *µ*m aufweist. Vorzugsweise ist das Einlassorgan ein Membranfilter oder Mehrschichtenfilter mit Membranfilter.

Bevorzugt kann über das Einlassorgan (4) das Kryoprotektivum in der gewünschten Menge zu einem Präparat, enthaltend das entnommene Nabelschnurblut, das Antikoagulanz und/oder gegebenenfalls ein Verdünnungsmittel zugesetzt werden. Bevorzugt wird das Kryoprotektivum in einer Gesamtkonzentration zwischen 1 und 15 Volumenprozent, besonders bevorzugt zwischen 2 und 10 Volumenprozent bezogen auf das Gesamtvolumen an entnommenem Nabelschnurblut zugesetzt. Als Kryoprotektivum eignet sich vorteilhaft Dimethylsulfoxid (DMSO).

Die Angaben in Volumenprozent (V%) einer zuzusetzenden Flüssigkeit leiten sich dabei aus dem Gesamtvolumen an entnommenem Nabelschnurblut (V) ab und ergeben sich als prozentualer Anteil zu diesem Wert (V%=*v*/V).

Vorteilhaft sind durch die Ausschlussgröße des Einlassorgans Reinstraumbedingungen für die Zufuhr eines Kryoprotektivums nicht erforderlich. Vorteilhaft kann das Zusetzen einer Flüssigkeit zu einem erfindungsgemäßen Blutbeutelsystems daher unter standardisierten hygienischen Bedingungen (bspw. Reinraum der Klasse D) erfolgen. Solche standardisierten hygienischen Bedingungen sind in allen Krankenhäusern vorhanden, wodurch besonders vorteilhaft ein zeit- und temperaturkritischer Individualtransport der einzelnen Nabelschnurblutproben in ein Zentral- und Sammellabor mit Reinstraumbedingungen entfällt. Ein Verlust von Blutstammzellen kann durch den Wegfall der Individualtransporte reduziert werden.

Zweckdienlich aber nicht notwendigerweise weist das erfindungsgemäße Blutbeutelsystem in der Peripherie des kombinierter Misch- und Einfrierbeutels (2) mindestens einen Satellitenbeutel auf, wobei der/die Satellitenbeutel über Verbindungsleitungen mit dem Blutbeutelsystem kommunizierend verbunden sind. Im Sinne der Erfindung sind "Satellitenbeutel" Leerbeutel, die bevorzugt ein Hohlvolumen zwischen 1 bis 20 mL, besonders bevorzugt zwischen 2 bis 15 mL aufweisen.

Vorteilhaft sind die Satellitenbeutel (6) so im erfindungsgemäßen Blutbeutelsystem integriert, dass eine einfache Entnahme von Nabelschnurblut und/oder Präparatproben aus dem kombinierten Misch-Einfrierbeutel (2) im Prozess der vorbereitenden Beuteleinlagerung möglich ist. Vorteilhafterweise ist das Blutbeutelsystem so konstruiert, dass eine separate Probenentnahme nach jedem Vorbereitungsschritt möglich ist.

Vorzugsweise sind die Satellitenbeutel (6) daher direkt an eine Hauptverbindungsleitung (12) angebunden, wodurch vorteilhaft die Anzahl an Zu- bzw. Abläufen an einem Misch- und Einfrierbeutel gering gehalten wird. Alternativ kann vorgesehen sein, dass die Satellitenbeutel direkt mit dem kombinierten Misch- und Einfrierbeutel (2) verbunden sind.
Bevorzugt dienen Satellitenbeutel der portionsweisen Auf- bzw. Entnahme von Nabelschnurblut oder Präparaten nach der Entnahme des Nabelschnurbluts, nach der Zugabe des Antikoagulanz oder nach der Zugabe des Kryoprotektivums. Vorteilhaft kann somit unabhängig vom Stadium der Weiterverarbeitung des Nabelschnurblutes im kombinierten Misch- und Einfrierbeutels eine Qualitätskontrolle des entnommenen Nabelschnurblutes bzw. der entsprechenden Produktgemische (Präparates) im geschlossenen System erfolgen, ohne das Hauptvolumen des entnommenen Nabelschnurblutes zu dekontaminieren. Folgende Qualitätsuntersuchungen werden üblicherweise am Präparat durchgeführt:
- Bestimmung der Zellzahl (bspw. mittels Durchflusszytometrie)
- Vitalität der Blutbestandteile
- gegebenenfalls Zellkulturnachweis (bei geringer Zellvitalität)
- Kontrolle der Sterilität des Präparats

Zur Bestimmung des Gesamtvolumens an entnommenem Nabelschnurblut (V) weisen die Satellitenbeutel vorteilhaft eine Füllstandsanzeige auf. Die Füllstandsanzeige kann durch Aufbringen entsprechender Markierungen auf der transparenten Beutelwand erfolgen, so dass insbesondere die Mindestfüllhöhe des entnommenen Nabelschnurblutes und die Sollfüllhöhe, die nach dem Auffüllen mit Flüssigkeit erreicht sein soll, kontrolliert werden können.

Zweckdienlich weist das erfindungsgemäße Blutbeutelsystem ein Auslassorgan (9) zur vollständigen und/oder portionsweisen Entnahme von Nabelschnurblut bzw. Präparaten des Nabelschnurbluts aus dem erfindungsgemäßen Blutbeutelsystem oder dessen Komponenten auf. Bevorzugt ist das Auslassorgan (9) direkt, d.h. unmittelbar an dem kombinierten Misch-und Einfrierbeutel lokalisiert.

Bevorzugt weist ein erfindungsgemäßes Blutbeutelsystem einen Injektionseinsatz (7) auf, der vorzugsweise an die Hauptverbindungsleitung (12) befestigt ist und mit dem Reservoirbereich (3) kommunizierend verbunden ist. Es kann vorgesehen sein, dass der Injektionseinsatz nach dem Einleiten von Flüssigkeiten, die die Kryokonservierung ermöglichen und/oder unterstützen, vom erfindungsgemäßen Blutbeutelsystem abgetrennt (bspw. abgeschweißt) wird.

In einer alternativ bevorzugten Ausgestaltung der Erfindung umfasst der Injektionseinsatz (7) ein penetrierbares Septum (nicht gezeigt), um die Injektion von Flüssigkeiten, die die Kryokonservierung ermöglichen und/oder unterstützen, insbesondere des Kryoprotektivums und/oder der Verdünnungsflüssigkeit durch das Septum in den Reservoirbereich (3) unter Verwendung einer hypodermischen Nadel und einer Spritze zu ermöglichen.
In einer bevorzugten erfindungsgemäßen Ausgestaltung ist der Reservoirbereich (3) zur Einlagerung von Flüssigkeiten, die die Kryokonservierung ermöglichen und/oder unterstützen, derart gestaltet, dass es vorteilhaft ist, die Flüssigkeiten, die die Kryokonservierung ermöglichen und/oder unterstützen, erst unmittelbar vor der Entnahme des Nabelschnurblutes über den Injektionseinsatz (7) durch die Verbindungsleitung in den Reservoirbereich einzuleiten. Vorteilhaft wird somit die innere Wandung der Hauptverbindungsleitung und daran anschließend die Wandung des Misch- und Einfrierbeutels (2) mit dem Antikoagulanz benetzt. Unmittelbar im Sinne der Erfindung bedeutet, dass das Einleiten der Flüssigkeit in einem Zeitraum von maximal 1 h, bevorzugt maximal 30 min vor der Entnahme des Nabelschnurblutes erfolgt.

In einer alternativ bevorzugten Ausgestaltung der Erfindung ist der Reservoirbereich (3) zur Einlagerung einer Flüssigkeiten, die die Kryokonservierung ermöglichen und/oder unterstützen, derart ausgebildet, dass es von Vorteil ist, die Flüssigkeiten, die die Kryokonservierung ermöglichen und/oder unterstützen, bereits vor der Sterilisation des erfindungsgemäßen Blutbeutelsystems in den Reservoirbereich (3) einzuleiten. Besonders bevorzugt ist der Reservoirbereich (3) hierin ein Sachet.

Im Sinne der Erfindung sind das sterilisierte Beutelsystem und die Komponenten des Beutelsystems zur einmaligen Nutzung bestimmt. Sogenannte medizinischen Einweg- bzw. Single-Use-Systeme und deren Komponenten besitzen eine Reihe von bekannten Vorteilen, wobei das Wissen über die nur einmalige Verwendung, wie beispielsweise der Vorrichtung zur Entnahme der Körperflüssigkeit, eine preiswerte Herstellung dieser Komponenten oder des gesamten Blutbeutelsystems erlauben. Weiterhin vorteilhaft erlaubt die Single-Use-Technologie eine einfache Modularisierung des Blutbeutelsystems, Flexibilität, geringe Umrüstzeiten und besonders vorteilhaft eine Minimierung von Kontaminationsrisiken.

Zweckdienlich befindet sich ein erfindungsgemäßes steriles Beutelsystem vor der Verwendung regelmäßig schon aus Lagerungsvorteilen in einer luftdichten vor Licht schützenden Umverpackung (10). Vorteilhaft dient die Umverpackung (10) dem Schutz vor mechanischen Beschädigungen. Bevorzugt besteht die Umverpackung (10) aus einer Kunststoff-Folie, die durch Verschweißen gasdicht verschlossen ist und als Dampfbarriere der Verdunstung des Antikoagulanz bei der Sterilisation entgegenwirkt.

Vorzugsweise weist eine Umverpackung eine Primärfolie aus *transparenten Folienmaterial* (bspw. PE- oder PP-basiert) auf, wobei vorgesehen ist, dass nur die Hauptverbindungsleitung (12) zugänglich ist, die das Punktionsstück (1) aufweist, wodurch vorteilhaft die restlichen Komponenten des erfindungsgemäßen Blutbeutelsystems bis zur Vorbereitung der Kryokonservierung in der Primärverpackung verbleiben. Vorteilhaft eignet sich das Material für die gewählte Sterilisationsmethode (bspw. Gamma-stabil, ETO-stabil).

Besonders bevorzugt enthält die Umverpackung zudem ein Folienbehältnis aus einem Licht- und Gasschutzmaterial (bspw. Aluminium-Verbundmaterial (als UV- und Verdunstungsschutz) und/oder eine sterilisierbare Transferverpackung (bspw. aus *Folie-Tyvek- oder Folie-Papier-Verbundmaterial* zum kontaminationsfreien Einschleusen in den OP-Bereich auf.

Es sei angemerkt, dass die erfindungsgemäßen Ausgestaltungen in jeder Anordnung miteinander kombinierbar sind.

Die Erfindung umfasst auch die Verwendung eines erfindungsgemäßen sterilen Blutbeutelsystems zur Aufnahme und Kryokonservierung von Nabelschnurblut, Knochenmark oder peripheren Blutstammzellen.

Vorteilhaft eignet sich das erfindungsgemäße sterile Blutbeutelsystem alternativ zur Einlagerung von menschlichen oder tierischen Zellpräparaten aller Art. Beispielsweise ist das Zellpräparat Knochenmark oder Blut. Das erfindungsgemäße Blutbeutelsystem ist grundsätzlich auch für die Aufnahme und/oder Einlagerung anderer Körperflüssigkeiten anwendbar. Besonders vorteilhaft dienen die Zellpräparate zur allogenen oder autologen therapeutischen und/oder kosmetischen Anwendung beim Menschen und/oder Tier.

### Ausführungsbeispiele

Weitere Merkmale und Vorteile der Erfindung ergeben sich aus den nachfolgenden schematischen Zeichnungen und Ausführungsbeispielen anhand derer die Erfindung beispielhaft näher erläutert werden soll, ohne die Erfindung auf diese zu beschränken.
Dabei zeigt:
**Fig. 1****:** eine schematische Aufsicht auf ein erfindungsgemäßes Blutbeutelsystem mit einem Sachet (3');
**Fig. 2****:** eine schematische Aufsicht auf eine alternative Ausführungsform eines erfindungsgemäßen Blutbeutelsystems mit zwei Satellitenbeuteln (6);
**Fig. 3****:** eine schematische Aufsicht auf eine weitere Ausführungsform eines erfindungsgemäßen Blutbeutelsystems mit Reservoirbereich (3) und Injektionsorgan (7);
**Fig. 4****:** Darstellung eines erfindungsgemäßen Blutbeutelsystems mit Umverpackung (10).
**Fig. 5****:** Separierter Misch- und Einfrierbeutel vor der Kryokonservierung.
**Fig. 6****:** eine schematische Aufsicht auf eine weitere Ausführungsform eines erfindungsgemäßen Blutbeutelsystems mit zwei Punktionsstücken (1)

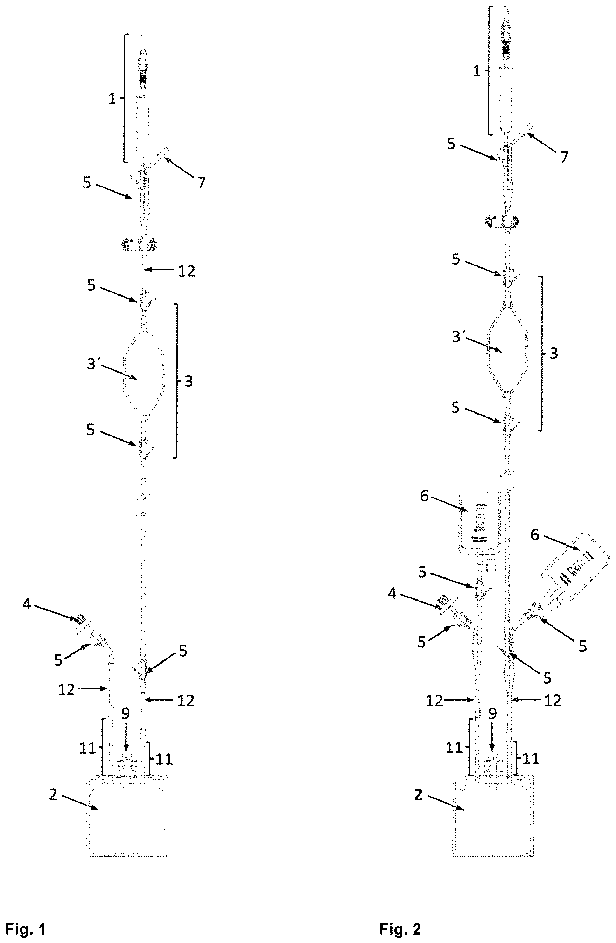

In **Fig. 1** ist eine schematische Aufsicht auf ein erfindungsgemäßes Blutbeutelsystemgezeigt. Das gezeigte Blutbeutelsystem umfasst dabei ein Punktionsstück (1), einen Reservoirbereich (3), der als Sachet (3') ausgebildet ist, einen kombinierten Misch- und Einfrierbeutel (2) und ein Einlassorgan (4). Die einzelnen Komponenten des Blutbeutelsystems sind durch Verbindungsleitungen miteinander verbunden und können durch Trennvorrichtungen (5) einzelnen voneinander separiert werden.

Die Beutelwände des kombinierten Misch- und Einfrierbeutel (2) bestehen aus Polyurethan und weisen eine Dicke von 0,25 mm auf. Der Misch- und Einfrierbeutel (2) ist als Einkammerbeutel ausgebildet und weist an einer Kante einen Einlass und einen Auslass auf, sodass der Misch- und Einfrierbeutel (2) über zwei Hauptverbindungsleitungen (12) mit den anderen Komponenten des Blutbeutelsystems kommunizierend verbunden ist. Die beiden Hauptverbindungsleitungen (12), die die einzelnen Komponenten miteinander verbinden, sind Schläuche aus Polyvinylchlorid (PVC), die einen Außendurchmesser von 4,2 mm und einen Innendurchmesser von 3,1 mm aufweisen.

Das Punktionsstück (1) ist hierbei eine Blutbeutelkanüle die einen Durchmesser von 2,2 mm und eine zentrale, in der Achse der Kanüle platzierte Öffnung (Backeye) aufweist. Die Kanüle kann von einer dampfsterilisierbaren Schutzkappe umschlossen werden. Die Kanüle kann nach Verwendung durch eine Schutzhülse gesichert werden.

Das Sachet (3') enthält 21 ml Zitratphosphatdextrose (CPD) als Antikoagulanz, wobei das CPD durch eine dem Sachet (3') vorgelagerte und eine nachgelagerte Herzklemme bis unmittelbar vor der Entnahme des Nabelschnurblutes im Sachet (3') verbleibt. Die Beutelwände des Sachets (3') weisen eine Dicke von 0,25 mm auf und bestehen aus einer PVC-Weichfolie die frei von Diethylhexylphthalat ist.

Unmittelbar vor der Entnahme des Nabelschnurblutes werden die den Reservoirbereich (3) begrenzenden Trennvorrichtungen (5) geöffnet, sodass das darin enthaltene CPD durch die Hauptverbindungsleitung (12) über den Einlass (11) in den kombinierten Misch- und Einfrierbeutel (2) geleitet wird.

Über das Punktionsstück (1) wird das Nabelschnurblut entnommen und in das erfindungsgemäße Blutbeutelsystem eingeleitet, wobei es, begünstigt durch die Schwerkraft, über die Hauptverbindungsleitung (12) durch den Reservoirbereich (3), der als Sachet (3') ausgebildet ist, in den kombinierten Misch- und Einfrierbeutel (2) fließt. Nach der Einleitung des Nabelschnurblutes in das Blutbeutelsystem erlaubt ein Membranfilter aus Nylon mit einer Ausschlussgröße von 0,22 *µ*m, der als Einlassorgan (4) ausgebildet ist, das Einleiten von Dimethylsulfoxid (DMSO) über eine zweite Hauptverbindungsleitung (12) (PVC-Schlauch) in den kombinierten Misch- und Einfrierbeutel (2).

Nach Abschluss dieser Präparation des entnommenen Nabelschnurblutes, wird der beladene Misch- und Einfrierbeutel (2) durch Abschweißen der beiden Einlässe (11) steril von den einzelnen Komponenten des Blutbeutelsystems separiert und kann bei -140°C eingelagert werden.

**Fig. 2** zeigt eine schematische Aufsicht auf eine alternative Ausführungsform eines erfindungsgemäßen Blutbeutelsystems, das aus einem Punktionsstück (1), einem Reservoirbereich (3), der als Sachet (3') ausgebildet ist, einem kombinierten Misch- und Einfrierbeutel (2) und zwei Satellitenbeuteln (6) gebildet wird. Neben einem Membranfilter (0,22 *µ*m) als Einlassorgan (4) für DMSO weist diese Ausgestaltung noch einen abgeschweißten Injektionseinsatz (7) auf, durch den während des Herstellungsverfahrens das CPD, das die Kryokonservierung unterstützt in den Reservoirbereich des Beutelsystems eingebracht worden ist. Die einzelnen Komponenten des Blutbeutelsystems sind durch Verbindungsleitungen (8), die als PVC Schläuche (4,2 mm/ 3,1 mm) ausgebildet sind, miteinander verbunden und können durch Trennvorrichtungen (5), die als Herzklemmen ausgebildet sind, einzeln voneinander separiert werden.

Die Satellitenbeutel (6) bestehen aus Diethylhexylphthalat-freiem PVC und weisen auf einer Beutelwand eine Füllstandsanzeige auf.

Unmittelbar vor der Entnahme des Nabelschnurblutes werden die den Reservoirbereich (3) begrenzenden Trennvorrichtungen (5) geöffnet, sodass das darin enthaltene CPD durch die Hauptverbindungsleitung (12) über den Einlass (11) in den kombinierten Misch- und Einfrierbeutel (2) geleitet wird, wodurch die Innenwandung der Hauptverbindungsleitung (12) und des kombinierten Misch- und Einfrierbeutels mit CPD benetzt werden. Die Entnahme des Nabelschnurblutes erfolgt über das Punktionsstück (1), wodurch es über die Hauptverbindungsleitung (12) durch den Reservoirbereich (3) und den Einlass (11) in den kombinierten Misch- und Einfrierbeutel (2) geleitet wird.

Nach der Entnahme des Nabelschnurblutes bzw. eines entsprechenden Produktgemisches (Präparat), enthaltend DMSO und/oder CPD, kann das jeweilige Präparat über eine Verbindungsleitung in einen Satellitenbeutel (6) geleitet werden. Die Satellitenbeutel können einzeln und steril von dem Blutbeutelsystem durch eine vorgelagerte Herzklemme abgetrennt werden.

Nach positiver Analyse der über die Satellitenbeutel (6) entnommenen Präparate, wird der beladene Misch- und Einfrierbeutel (2) durch Abschweißen der beiden Einlässe (11) steril von den einzelnen Komponenten des Blutbeutelsystems separiert und kann bei -140°C in der N₂-Gasphase eingelagert werden.

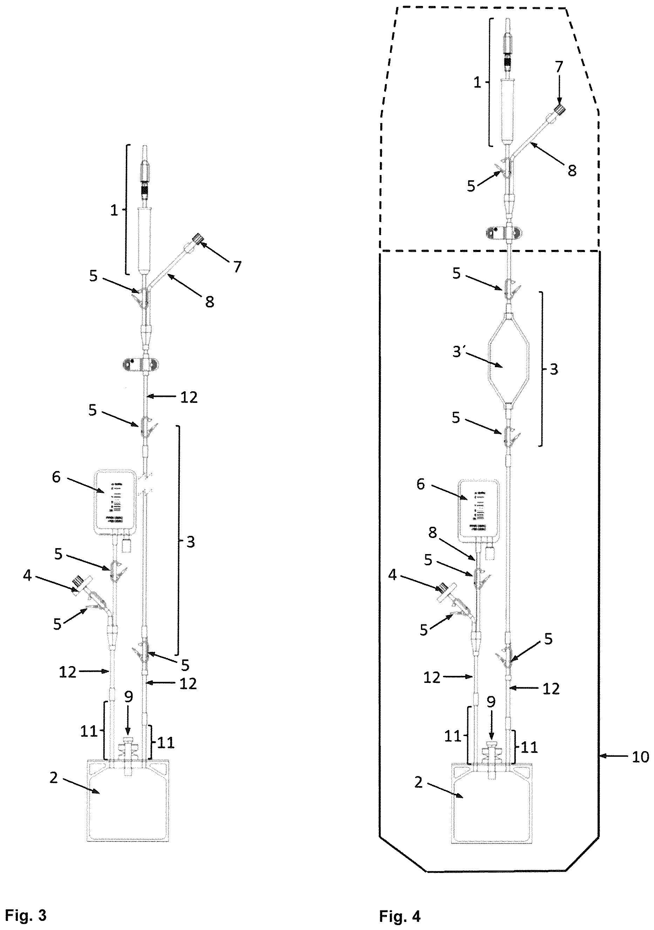

**Fig. 3** ist eine schematische Aufsicht auf eine weitere Ausführungsform eines erfindungsgemäßen Blutbeutelsystems mit einem Injektionseinsatz (7). Das Blutbeutelsystem umfasst ein Punktionsstück (1), einen kombinierten Misch- und Einfrierbeutel (2) und einen Satellitenbeutel (6), wobei der Reservoirbereich (3) durch die Hauptverbindungsleitung zwischen Entnahmeadapter (1) und dem Misch- und Einfrierbeutel (2) gebildet wird. Neben einem Membranfilter (0,22 *µ*m) als Einlassorgan (4) weist diese Ausgestaltung noch zusätzlich ein Injektionseinsatz (7) mit Septum auf, durch den Flüssigkeiten, die die Kryokonservierung unterstützen in das Beutelsystem eingebracht werden können.

Über den Injektionseinsatz (7) kann der Reservoirbereich (3) vor der Entnahme des Nabelschnurblutes direkt mit einem Antikoagulanz (bspw. CPDA) beladen werden, wobei erst nach dem Öffnen der dem Reservoirbereich nachgeordneten Trennvorrichtung (5), die als Herzklemme ausgebildet ist, das Antikoagulanz in den Misch- und Einfrierbeutel eingeleitet wird.

Die Entnahme und das Einleiten des Nabelschnurblutes über den Reservoirbereich in den kombinierten Misch- und Einfrierbeutel (2) erfolgt über das Punktionsstück (1). Nach der Zugabe von DMSO zu dem entnommenen Nabelschnurblut im Misch- und Einfrierbeutel (2) über das Einlassorgan (4) und die zweite Hauptverbindungsleitung, kann ein kleines Volumen in den Satellitenbeutel (6) geleitet werden, sodass nach Separierung des Satellitenbeutels (6) das darin enthaltene Präparat auf dessen Zellvitalität analysiert werden kann.

Nach positiver Analyse der über die Satellitenbeutel (6) entnommenen Präparate, wird der beladene Misch- und Einfrierbeutel (2) durch Abschweißen der beiden Einlässe (11) steril von den einzelnen Komponenten des Blutbeutelsystems separiert und kann bei -140°C in der N₂-Gasphase eingelagert werden.

**Fig. 4** zeigt eine schematische Aufsicht auf ein erfindungsgemäßes Blutbeutelsystem mit einem Punktionsstück (1) und einem kombinierten Misch- und Einfrierbeutel (2) die über eine Hauptverbindungsleitung (12) kommunizierend miteinander verbunden sind, wobei der zwischengeordnete Reservoirbereich (3) als Sachet (3') ausgebildet ist. Alle Komponenten des Blutbeutelsystems sind kommunizierend durch zwei Hauptverbindungsleitungen (8) und eine nebengeordnete Verbindungsleitung, die den Satellitenbeutel (6) mit dem Beutelsystem kommunizierend verbindet, miteinander verbunden. Die Umverpackung (10) aus einer Primärfolie aus und dient dem Schutz vor mechanischen Beschädigungen während der Lagerung und dem Transport des Blutbeutelsystems. Die gestrichelte Linie verdeutlicht, dass die Primärfolie derart geöffnet werden kann, dass eine Hauptverbindungsleitung (12) mit dem daran gekoppelte Punktionsstück (1) unabhängig von den restlichen Komponenten des Blutbeutelsystems zugänglich sind.

Unmittelbar vor der Entnahme des Nabelschnurblutes werden die den Reservoirbereich (3) begrenzenden Trennvorrichtungen (5) geöffnet, sodass das darin enthaltene Kryoprotektivum (bspw. CPD, CPDA) durch die Hauptverbindungsleitung (12) über den Einlass (11) in den kombinierten Misch- und Einfrierbeutel (2) geleitet wird, wodurch die Innenwandung der Hauptverbindungsleitung (12) und des kombinierten Misch- und Einfrierbeutels mit CPD benetzt werden.

Die Entnahme des Nabelschnurblutes erfolgt über das freigelegte Punktionsstück (1), wodurch es über die Hauptverbindungsleitung (12) durch den Reservoirbereich (3) und den Einlass (11) in den kombinierten Misch- und Einfrierbeutel (2) geleitet wird.

**Fig. 5** zeigt eine schematische Aufsicht des separierten Misch- und Einfrierbeutels (2) wie er für die Einlagerung verwendet wird. Der Beutel ist vollständig von den anderen Komponenten des Blutbeutelsystems separiert. Die Einlässe des kombinierten Misch-und Einfrierbeutels (11) sind abgeschweißt.

**Fig. 6** zeigt eine schematische Aufsicht auf eine alternative Ausführungsform eines erfindungsgemäßen Blutbeutelsystem, das aus zwei Punktionsstücken (1), einem Reservoirbereich (3), der als Sachet (3') ausgebildet ist, einem kombinierten Misch- und Einfrierbeutel (2) und zwei Satellitenbeuteln (6) gebildet wird. Durch die zweite Punktion kann ggf. die Ausbeute an verwertbarem Nabelschnurblut gesteigert werden, da somit an mehreren Positionen Nabelschnurblut entnommen werden kann.

### Bezugszeichenliste

- **(1)**: Punktionsstück
- **(2)**: kombinierter Misch- und Einfrierbeutel
- **(3)**: zwischengeordneter Reservoirbereich
- **(4)**: Einlassorgan
- **(5)**: Trennvorrichtung
- **(6)**: Satellitenbeutel
- **(7)**: Injektionseinsatz
- **(8)**: Verbindungsleitungen
- **(9)**: Auslassorgan
- **(10)**: Umverpackung
- **(11)**: Einlass des kombinierten Misch- und Einfrierbeutels
- **(12)**: Hauptverbindungsleitung

## Patentansprüche

1. Steriles Blutbeutelsystem für die Entnahme und Kryokonservierung von Nabelschnurblut, das als Komponenten zumindest ein Punktionsstück (1), einen kombinierten Misch-und Einfrierbeutel (2) und ein Einlassorgan (4) aufweist, **dadurch gekennzeichnet, dass** die einzelnen Komponenten des Blutbeutelsystems untereinander kommunizierend über Verbindungsleitungen (8) miteinander so verbunden sind, dass der kombinierte Misch-und Einfrierbeutel (2) zur direkten Aufnahme von Nabelschnurblut während dessen Entnahme dient und gleichzeitig zur Kryokonservierung nutzbar ist, dass die einzelnen Komponenten des Blutbeutelsystems durch Trennvorrichtungen (5) steril voneinander separierbar sind, dass dem Punktionsstück (1) und dem Misch- und Einfrierbeutel (2) ein Reservoirbereich (3) zwischengeordnet ist, wobei der Reservoirbereich (3) an seinen gegenüberliegenden Seiten jeweils eine Öffnung, einen Ein- und Auslass, aufweist, wobei der Reservoirbereich (3) in die Hauptverbindungsleitung (12), die das Punktionsstück (1) und den kombinierten Misch- und Einfrierbeutel (2) verbindet, direkt und gleichgerichtet integriert ist.

2. Blutbeutelsystem nach Anspruch 1, **dadurch gekennzeichnet, dass** der kombinierte Misch- und Einfrierbeutel (2) ein Einkammerbeutel oder Mehrkammerbeutel ist.

3. Blutbeutelsystem nach einem der Ansprüche 1 oder 2, **dadurch gekennzeichnet, dass** der kombinierte Misch- und Einfrierbeutel (2) maximal zwei Einlässe (11) aufweist, wobei der kombinierte Misch- und Einfrierbeutel (2) über daran gekoppelte Hauptverbindungsleitungen (12) kommunizierend mit dem Punktionsstück (1) und dem Einlassorgan (4) kommunizierend verbunden ist.

4. Blutbeutelsystem nach einem der Ansprüche 1 bis 3, **dadurch gekennzeichnet, dass** die Beutelwände des kombinierten Misch- und Einfrierbeutels (2) aus einem kältebeständigen Organopolymer bestehen, wobei das Organopolymer eine O₂-Permeabilität im Bereich zwischen 1500 und 2500 cm³ pro m² Organopolymer aufweist.

5. Blutbeutelsystem nach einem der Ansprüche 1 bis 4, **dadurch gekennzeichnet, dass** das Blutbeutelsystem einen Injektionseinsatz (7) aufweist, welcher an die Hauptverbindungsleitung (12) befestigt und mit dem Reservoirbereich (3) kommunizierend verbunden ist.

6. Blutbeutelsystem nach einem der Ansprüche 1 bis 5, **dadurch gekennzeichnet, dass** der Reservoirbereich (3) ein Antikoagulanz und/oder ein Kryoprotektivum und/oder eine Verdünnungsflüssigkeit enthält.

7. Blutbeutelsystem nach einem der Ansprüche 1 bis 6, **dadurch gekennzeichnet, dass** der Reservoirbereich (3) als Sachet (3') ausgebildet ist.

8. Blutbeutelsystem nach einem der Ansprüche 1 bis 7, **dadurch gekennzeichnet, dass** in der Peripherie des kombinierten Misch- und Einfrierbeutels (2) mindestens ein Satellitenbeutel angeordnet ist, wobei dieser über Verbindungsleitungen (8) kommunizierend mit dem Blutbeutelsystem verbunden ist.

9. Verwendung eines sterilen Blutbeutelsystems nach einem der Ansprüche 1 bis 8 zur Aufnahme und Kryokonservierung von Nabelschnurblut, Knochenmark oder peripheren Blutstammzellen.

## Claims

1. Sterile blood bag system for taking and cryopreserving umbilical cord blood, which system comprises at least one puncture part (1), a combined mixing and freezing bag (2) and an inlet element (4) as components, **characterised in that** the individual components of the blood bag system are interconnected so as to communicate with one another via connection lines (8) in such a way that the combined mixing and freezing bag (2) can be used for directly receiving blood from an umbilical cord during the taking thereof and can simultaneously be used for cryopreservation, **in that** the individual components of the blood bag system can be sterilely separated from one another by separating devices (5), **in that** a reservoir region (3) is arranged between the puncture part (1) and the mixing and freezing bag (2), the reservoir region (3) comprising an opening, an inlet and outlet, on each opposing side, the reservoir region (3) being integrated, in a direct and aligned manner, into the main connection line (12) that connects the puncture part (1) and the combined mixing and freezing bag (2).

2. Blood bag system according to claim 1, **characterised in that** the combined mixing and freezing bag (2) is a single-chamber bag or a multi-chamber bag.

3. Blood bag system according to either claim 1 or claim 2, **characterised in that** the combined mixing and freezing bag (2) comprises a maximum of two inlets (11), the combined mixing and freezing bag (2) being communicatively connected to the puncture part (1) and the inlet element (4) so as to communicate via main connection lines (12) coupled thereto.

4. Blood bag system according to any of claims 1 to 3, **characterised in that** the bag walls of the combined mixing and freezing bag (2) consist of a cold-resistant organopolymer, the organopolymer having an O₂ permeability in the range between 1500 and 2500 cm³ per m² of organopolymer.

5. Blood bag system according to any of claims 1 to 4, **characterised in that** the blood bag system comprises an injection insert (7) which is fastened to the main connection line (12) and communicatively connected to the reservoir region (3).

6. Blood bag system according to any of claims 1 to 5, **characterised in that** the reservoir region (3) contains an anticoagulant and/or a cryoprotectant and/or a thinning liquid.

7. Blood bag system according to any of claims 1 to 6, **characterised in that** the reservoir region (3) is designed as a sachet (3').

8. Blood bag system according to any of claims 1 to 7, **characterised in that** at least one satellite bag is arranged on the periphery of the combined mixing and freezing bag (2), said satellite bag being connected to the blood bag system so as to communicate via connection lines (8).

9. Use of a sterile blood bag system according to any of claims 1 to 8 for taking and cryopreserving umbilical cord blood, bone marrow or peripheral blood stem cells.

## Revendications

1. Système de poche de sang stérile pour le prélèvement et la cryoconservation du sang de cordon ombilical, qui présente comme composants au moins une pièce de ponction (1), une poche de mélange et de congélation (2) combinée et un organe d'entrée (4), **caractérisé en ce que** les différents composants du système de poche de sang sont raccordés les uns aux autres de façon communicante entre eux par le biais de conduites de raccordement (8) de telle sorte que la poche de mélange et de congélation (2) combinée sert à la réception directe de sang de cordon ombilical pendant son prélèvement et est en même temps utilisable pour la cryoconservation, **en ce que** les différents composants du système de poche de sang peuvent être séparés les uns des autres de façon stérile par des dispositifs de séparation (5), **en ce qu'**une zone de réservoir (3) est interposée entre la pièce de ponction (1) et la poche de mélange et de congélation (2), la zone de réservoir (3) présentant, sur ses côtés opposés, respectivement une ouverture, une entrée et une sortie, la zone de réservoir (3) étant intégrée directement et dans le même sens dans la conduite de raccordement principale (12) qui raccorde la pièce de ponction (1) et la poche de mélange et de congélation (2) combiné.

2. Système de poche de sang selon la revendication 1, **caractérisé en ce que** la poche de mélange et de congélation (2) combinée est une poche à une chambre ou une poche à plusieurs chambres.

3. Système de poche de sang selon l'une des revendications 1 ou 2, **caractérisé en ce que** la poche de mélange et de congélation (2) combinée présente au maximum 2 entrées (11), la poche de mélange et de congélation (2) combinée étant, par le biais de conduites de raccordement principales (12) qui sont couplées avec elle de façon communicante, raccordée de façon communicante à la pièce de ponction (1) et à l'organe d'entrée (4).

4. Système de poche de sang selon l'une des revendications 1 à 3, **caractérisé en ce que** les parois de poche de la poche de mélange et de congélation (2) combinée sont composées d'un organopolymère résistant au froid, l'organopolymère présentant une perméabilité à l'O₂ dans la plage entre 1 500 et 2 500 cm³ par m² d'organopolymère.

5. Système de poche de sang selon l'une des revendications 1 à 4, **caractérisé en ce que** le système de poche de sang présente un embout d'injection (7) qui est fixé à la conduite de raccordement principale (12) et est raccordé de façon communicante à la zone de réservoir (3).

6. Système de poche de sang selon l'une des revendications 1 à 5, **caractérisé en ce que** la zone de réservoir (3) contient un anticoagulant et/ou un cryoprotecteur et/ou un liquide de dilution.

7. Système de poche de sang selon l'une des revendications 1 à 6, **caractérisé en ce que** la zone de réservoir (3) est constituée sous forme de sachet (3').

8. Système de poche de sang selon l'une des revendications 1 à 7, **caractérisé en ce que**, dans la périphérie de la poche de mélange et de congélation (2) combinée, il est disposé au moins une poche satellite, celle-ci étant raccordée de façon communicante au système de poche de sang par le biais de conduites de raccordement (8).

9. Utilisation d'un système de poche de sang stérile selon l'une des revendications 1 à 8 pour la réception et la cryoconservation de sang de cordon ombilical, de moelle osseuse ou de cellules souches de sang périphérique.
